# EUROPÄISCHE PATENTSCHRIFT

(11) **EP 0 992 477 B1**
(45) Veröffentlichungstag und Bekanntmachung des Hinweises auf die Patenterteilung: **05.02.2003**
(21) Anmeldenummer: 99118458.1
(22) Anmeldetag: 17.09.1999
(51) Int. Cl.: C07C 45/51, C07C 47/21

(54) **Verfahren zur Herstellung von Citral**
Process for the preparation of citral
Procédé de préparation de citral

(30) Priorität: 07.10.1998 DE 19846056
(43) Veröffentlichungstag der Anmeldung: 12.04.2000
(73) Patentinhaber: BASF AKTIENGESELLSCHAFT, 67056 Ludwigshafen (DE)
(72) Erfinder: Therre, Jörg, Dr., 67550 Worms (DE); Kaibel, Gerd, Dr., 68623 Lampertheim (DE); Aquila, Werner, Dr., 68309 Mannheim (DE); Wegner, Günter, Dr., 67354 Römerberg (DE); Fuchs, Hartwig, 67063 Ludwigshafen (DE)

(56) Entgegenhaltungen:
- EP-A- 0 021 074

## Beschreibung

Die vorliegende Erfindung betrifft ein verbessertes Verfahren zur Herstellung von 3,7-Dimethyl-octa-2,6-dien-1-al (Citral) durch thermische Spaltung von 3-Methyl-2-buten-1-al-diprenylacetal.

Diese Reaktion ist, sieht man von den erfindungsgemäßen Verbesserungen ab, in ihren wesentlichen Merkmalen schon u.a. aus der DE 24 11 530 und EP 21 074 B1 bekannt.

Es handelt sich hierbei um eine recht komplexe Umsetzung, die - wie aus folgendem Reaktionsschema hervorgeht - über drei Stufen verläuft.
1. Thermische Spaltung des Acetals der Formel II in 3-Methyl-2-buten-1-ol (Prenol) der Formel III und den cis/trans-Prenyl-(3-methyl-butadienyl)-ether (Dienylether) der Formel IV 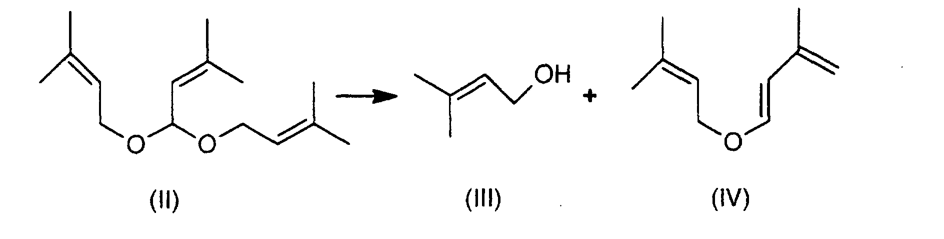
2. Thermische Umlagerung des Dienylethers der Formel IV in einer Claisen-Umlagerung zu dem 2,4,4-Trimethyl-3-formyl-1,5-hexadien (Formylhexadien) der Formel V 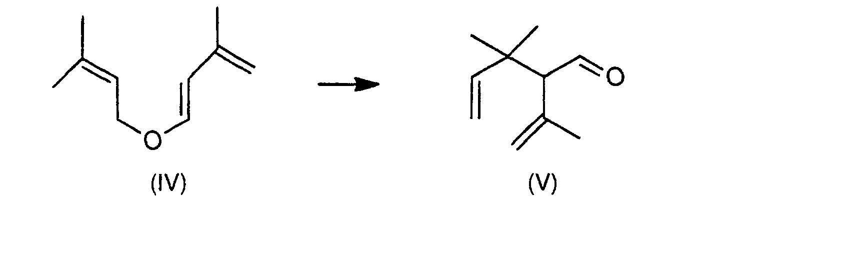 und
3. Thermische Umlagerung des Dienylethers der Formel V in einer Cope-Umlagerung zu dem gewünschten Citral der Formel I

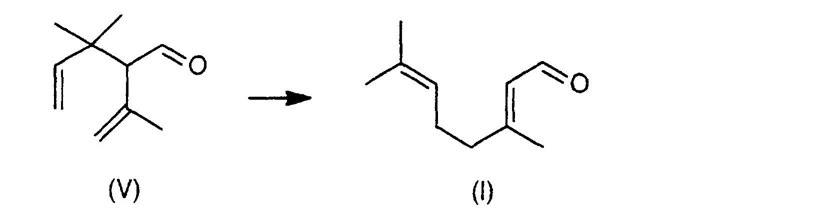

Citral wird als Duftstoff verwendet und ist darüber hinaus als Ausgangsstoff für andere Duftstoffe, wie das Geraniol, sowie als Ausgangsstoff für Vitamine, beispielsweise das Vitamin A, von großer Bedeutung.

Da sowohl die Ausgangsverbindung der Formel II als auch das gewünschte Citral, das Nebenprodukt Prenol und die Citralvorstufen der Formeln IV und V empfindliche Substanzen sind, die in unerwünschter Weise weiter reagieren können, gelang die Herstellung des Citrals zunächst nur mit Ausbeuten von 60 bis 70 %. Weitaus bessere Ausbeuten an Citral erhielt man auch in industriellem Maßstab gemäß dem Verfahren der EP 0 021 074 B1. In dieser Europäischen Patentschrift wird ein Verfahren zur Herstellung des Citrals aus dem Acetal der Formel II beschrieben, das dadurch gekennzeichnet ist, daß man das als Nebenprodukt gebildete Prenol während der Reaktion fortlaufend aus dem Reaktionsgemisch abdestilliert. Das Citral und die Citralvorstufen der Formeln IV und V werden dabei mittels einer Destillationskolonne in der Reaktionsmischung zurückgehalten. Die fortlaufende Entfernung des Prenols aus der Reaktionsmischung bewirkte eine Steigerung der Citralausbeute auf etwa 85 bis 90 %. Zur weiteren Steigerung der Ausbeute an Citral wurde in EP 0 021 074 zusätzlich die Mitverwendung einer Substanz mit einem Siedepunkt zwischen dem von Prenol und dem von Citral und den Citralvorstufen der Formeln IV und V (dem sogenannten Zwischensieder) empfohlen. Durch diese Maßnahme konnte die Citralausbeute auf ca. 95 % der Theorie erhöht werden.

Nachteilig an dem beschriebenen, an sich sehr guten Verfahren ist, daß das Verfahren nur zur diskontinuierlichen Verfahrensführung geeignet ist und lange Verweilzeiten (etwa 6 Stunden) und daher große Apparaturen erfordert. Ein weiterer Nachteil des bekannten Verfahrens ist, daß in das System ein Hilfsstoff, nämlich der Zwischensieder, eingebracht werden muß, was einen zusätzlichen Aufwand für die Dosierung des Hilfsstoffes, für die Überwachung der Konzentration des Hilfsstoffes und für das Abtrennens des Hilfsstoffes bedeutet.

Es war daher die Aufgabe der Erfindung, das Verfahren zur Herstellung von Citral durch thermische Spaltung des Acetals der Formel II so zu verbessern, daß die oben beschriebenen Nachteile vermieden werden. Dies bedeutete, das Verfahren so zu verbessern, daß die Verwendung von Hilfsstoffen nicht erforderlich ist, daß mit kürzeren Verweilzeiten und daher in kleinen Apparaturen gearbeitet werden kann und dennoch hohe Selektivitäten an Citral erzielt werden.

Gegenstand der Erfindung ist dementsprechend ein Verfahren zur Herstellung von 3,7-Dimethyl-2,6-octadien-1-al (Citral) der Formel I 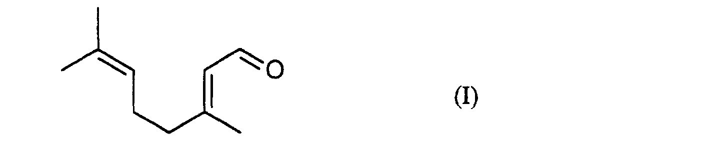 durch thermische Spaltung ggf. in Gegenwart eines sauren Katalysators von 3-Methyl-2-buten-1-al-diprenylacetal der Formel II 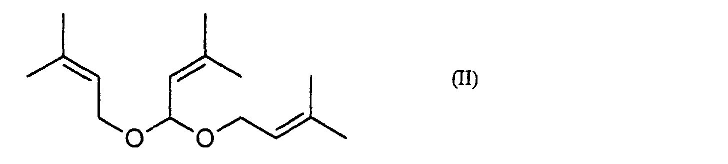 unter Abspaltung von 3-Methyl-2-buten-1-ol (Prenol) der Formel III zum cis/trans-Prenyl-(3-methyl-butadienyl)-ether der Formel IV

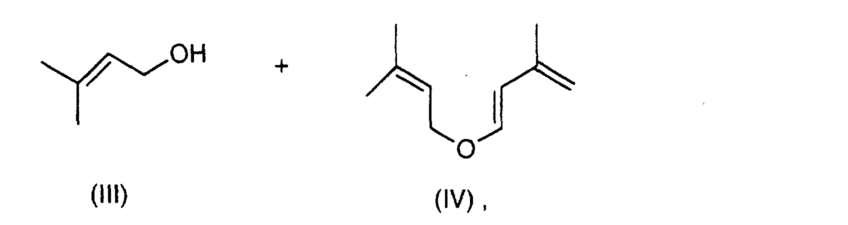

Claisen-Umlagerung des erhaltenen Butadienylethers der Formel IV zu 2,4,4-Trimethyl-3-formyl-1,5-hexadien der Formel V 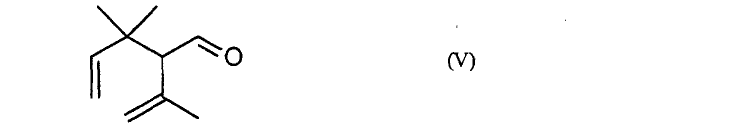 und anschließende Cope-Umlagerung desselben zum Citral der Formel I, das dadurch gekennzeichnet ist, daß man sowohl das gebildete Prenol der Formel III, als auch die intermediär gebildeten Zwischenprodukte der Formeln IV und V und das Citral schon während der Umsetzung fortlaufend destillativ aus dem Reaktionsgemisch entfernt und die Zwischenprodukte der Formeln IV und V vor oder nach destillativer Abtrennung von Prenol und ggf. Nebenprodukten durch Erhitzen auf Temperaturen zwischen 100 und 200°C in Citral umlagert.

Mit besonderem Vorteil gelingt das erfindungsgemäße Verfahren, wenn man die thermische Spaltung des Acetals der Formel II im unteren Teil oder im Sumpf einer als Spaltkolonne funktionierenden Destillationskolonne mit 5 bis 100 theoretischen Trennstufen durchführt.

Hierbei hat es sich als sehr zweckmäßig erwiesen, wenn man das Acetal der Formel II und ggf. den sauren Katalysator in den unteren Teil der Destillationskolonne, in den Sumpf der Destillationskolonne oder in den Verdampfer (2) der Destillationskolonne einbringt.

Eine Möglichkeit zur Durchführung des erfindungsgemäßen Verfahrens ist dadurch gekennzeichnet, daß man die thermische Spaltung des Acetals der Formel II im unteren Teil oder im Sumpf einer Destillationskolonne mit 5 bis 100 theoretischen Trennstufen durchführt, wobei man das Acetal der Formel II durch geeignete Auswahl der Destillationsbedingungen im unteren Teil bzw. im Sumpf der Kolonne hält, das gebildete Citral der Formel I, die intermediär gebildeten Zwischenprodukte der Formeln IV und V und das abgespaltene Prenol der Formel III gemeinsam am Kopf der Kolonne als Kopfstrom gewinnt und das Gemisch aus Citral und den Zwischenprodukten der Formeln IV und V vor oder nach destillativer Abtrennung von Prenol und ggf. Nebenprodukten durch ein beheiztes Verweilzeitrohr (7) führt, in dem die Zwischenprodukte der Formeln IV und V bei Temperaturen von 100 bis 200°C zu Cital umgelagert werden.
Mit besonderem Vorteil gestaltet sich das erfindungsgemäße Verfahren, wenn man die thermische Spaltung des Acetals der Formel II im unteren Teil (9) oder im Sumpf (10) einer Destillationskolonne (1) mit 5 bis 100 theoretischen Trennstufen durchführt, wobei man das Acetal der Formel II durch geeignete Auswahl der Destillationsbedingungen im unteren Teil (9) bzw. im Sumpf (10) der Kolonne (1) hält, das gebildete Citral der Formel I und die intermediär gebildeten Zwischenprodukte der Formeln IV und V gemeinsam an einem im mittleren oder unteren Teil der Kolonne angeordneten Seitenabzug (5) flüssig oder dampfförmig abzieht und das abgespaltene Prenol der Formel III am Kopf (8) der Kolonne mit dem Kopfstrom abtrennt.

Das an dem Seitenabzug (5) abgezogene oder aber aus dem Kopfstrom einer Kolonne ohne Seitenabzug gewonnene Gemisch aus Citral und den intermediär gebildeten Zwischenprodukten der Formeln IV und V führt man anschließend zweckmäßig durch ein beheiztes Verweilzeitrohr (7), in dem die Zwischenprodukte der Formeln IV und V bei Temperaturen von 100 bis 200 °C zu Citral umgelagert werden. Das als Ausgangsverbindung benötigte Acetal kann, wie beispielsweise in EP 0 021 074 B1 beschrieben, recht einfach durch Umsetzung von 3-Methyl-2-buten-1-ol (Prenol) mit 3-Methyl-2-buten-1-al (Prenal) hergestellt werden. Abhängig von den Herstellungsbedingungen des Acetals kann dieses zwischen 0,1 und 30 Gew.-% an nicht umgesetztem Prenal und zwischen 0,1 und 60 Gew.-% an nicht umgesetztem Prenol enthalten. Vorteilhaft für das erfindungsgemäße Verfahren ist es, wenn die Konzentration des eingesetzten Acetals der Formel II im Ausgangsmaterial über 30 Gew.-%, vorzugsweise über 70 Gew.-%, liegt.

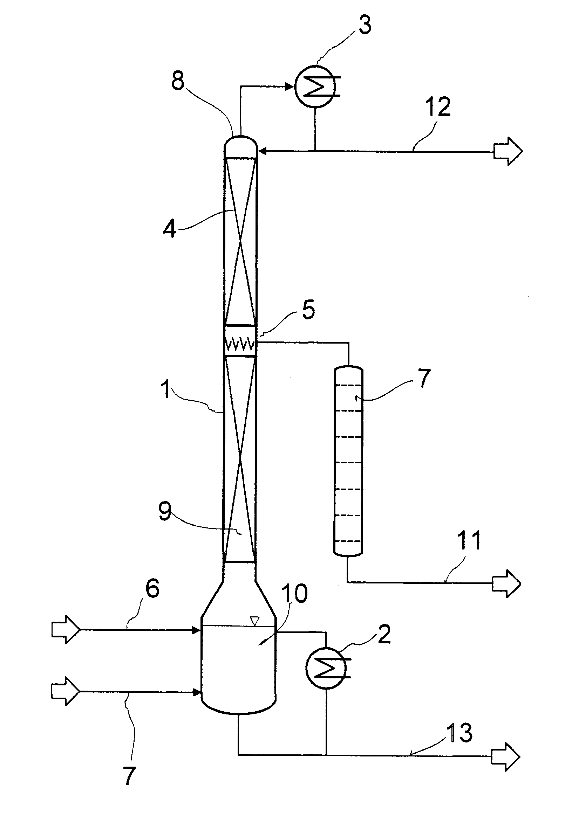

Die bevorzugte Durchführung des erfindungsgemäßen Verfahrens wird anhand der beigefügten Figur nachfolgend beschrieben:

Die Spaltung des Acetals der Formel II zu Prenol, Citral und den Citralvorstufen der Formeln IV und V erfolgt in einer Destillationskolonne, der sogenannten Spaltkolonne (1), die mit einem Verdampfer (2) und einem Kondensator (3) ausgestattet ist. Als Einbauten (4) für die Kolonne eignen sich Böden, Füllkörper und insbesondere strukturierte Packungen aus Blech (z.B. Sulzer 250.Y) oder Metallgewebe (z.B. Sulzer BX oder Sulzer CY). Die Zahl der theoretischen Trennstufen der Kolonne soll zwischen 5 und 100 betragen. In einer bevorzugten Ausführung der Erfindung ist ein Seitenabzug (5) vorhanden, aus dem das Citral und die Citralvorstufen in dampfförmiger oder flüssiger Form aus der Kolonne entnommen werden. Der Seitenabzug (5) befindet sich im mittleren oder unteren Teil der Kolonne (1), wobei der Seitenabzug (5) zweckmäßig zwischen 2 und 20 theoretische Trennstufen oberhalb der Zugabestelle (6) des Acetals liegt. Oberhalb des Seitenabzugs (5) befinden sich noch zwischen 2 und 80 theoretische Trennstufen. Das Gemisch aus Citral und den Citralvorstufen wird durch ein beheiztes Verweilzeitrohr (7) geführt, in dem die Citralvorstufen der Formeln IV und V thermisch zu Citral umgelagert werden. Das gewonnene Citral kann dann an der Entnahmestelle (11) entnommen werden.

Das aus dem Kopfstrom im Kondensator (3) zurückgewonnene Prenol und ggf. 3-Methyl-2-buten-1-al können an der Entnahmestelle (12) entnommen und wieder zur Herstellung des Acetals der Formel II in den Prozeß zurückgeführt werden. Die bei dem Verfahren anfallenden relativ geringen Mengen an Sumpfablauf können an der Entnahmestelle (13) entfernt werden.

Das Acetal wird im unteren Teil der Spaltkolonne (1), bevorzugt aber in den Sumpf (10) der Spaltkolonne (1) oder in den Verdampfer (2) zugegeben. Gegebenenfalls kann der Sumpf (10) der Spaltkolonne durch einen Behälter vergrößert sein, um ein größeres Reaktionsvolumen zur Verfügung zu haben. Der Zufluß an dem Acetal der Formel II wird zweckmäßig so bemessen, daß die auf den Zufluß an Acetal bezogene Verweilzeit zwischen einer Minute und 6 Stunden, vorzugsweise zwischen 5 Minuten und 2 Stunden beträgt. Diese relativ kurze Verweilzeit und die dadurch bewirkten kleinen kostengünstigeren Apparaturen stellen einen deutlichen Vorteil des erfindungsgemäßen Verfahrens dar.

Das Verfahren kann prinzipiell diskontinuierlich, halbkontinuierlich oder kontinuierlich durchgeführt werden. Für die halbkontinuierliche Verfahrensführung wird zu Beginn der Umsetzung eine gewisse Menge an Acetal vorgelegt und dann gegebenenfalls weiteres Acetal zudosiert.

Mit besonderem Vorteil führt man das Verfahren aber kontinuierlich durch.

Gewünschtenfalls kann zum Anfahren der Apparatur im Sumpf der Kolonne eine schwersiedende inerte Verbindung vorgelegt werden, um einen Mindestfüllstand des Sumpfes und des Verdampfers sicherzustellen. Geeignet sind alle unter den Reaktionsbedingungen inerten flüssigen Stoffe, die einen höheren Siedepunkt als Citral und insbesondere als das Acetal der Formel II besitzen, wie zum Beispiel die Kohlenwasserstoffe Tetradecan, Pentadecan, Hexadecan, Octadecan, Eicosan, oder Ether, wie Diethylenglycoldibutylether, Weißöle, Parafinöle oder Mischungen der genannten Verbindungen.

Die Umsetzung kann ohne Katalysator, also nur durch Erhitzen, durchgeführt werden. Mit besonderem Vorteil wird sie jedoch in Gegenwart eines sauren Katalysators vorgenommen. Als saure Katalysatoren sind hierbei insbesondere nicht flüchtige Protonensäuren wie Schwefelsäure, p-Toluol-sulfonsäure und vor allem Phosphorsäure geeignet.

Der Katalysator wird mit Vorteil in den unteren Teil der Spaltkolonne (1), vorzugsweise in den Sumpf der Spaltkolonne oder in den Verdampfer (2) zugegeben.

Die Konzentration des Katalysators im Reaktionsgemisch beträgt zweckmäßig zwischen 0,0001 Gew.-% und 1 Gew.-%, vorzugsweise 0,0005 und 0,5 Gew.-%, bezogen auf die Gesamtmenge des Reaktionsgemisches. Der Katalysator kann in reiner Form oder gelöst in einem geeigneten Lösungsmittel zugegeben werden. Als Lösungsmittel für den Katalysator sind Wasser, Alkohole und Kohlenwasserstoffe, vorzugsweise aber die im Reaktionsgemisch vorhandenen Verbindungen, wie Prenol, 3-Methyl-2-butenal, Citral, die Citralvorstufen der Formeln IV und V und insbesondere das Acetal der Formel II geeignet. Wird Wasser verwendet, so soll dessen Konzentration im Reaktionsgemisch möglichst niedrig sein, da Wasser die Rückreaktion des Acetals zu Prenol und 3-Methyl-2-butenal bewirkt. Bei den übrigen Lösungsmitteln ist es zweckmäßig, wenn die Konzentration des Katalysators im Lösungsmittel zwischen 0,01 Gew.-% und 50 Gew.-% liegt.

Der Druck im Sumpf der Spaltkolonne beträgt mit Vorteil zwischen 1 mbar und 100 mbar, insbesondere zwischen 10 und 70 mbar. Die Temperatur im Sumpf der Kolonne liegt im allgemeinen zwischen 70°C und 270°C, vorzugsweise zwischen 100°C und 220°C, insbesondere zwischen 130°C und 190°C.

Das Rückflußverhältnis am Kopf der Spaltkolonne liegt mit Vorteil zwischen 0,5 und 70, insbesondere zwischen 2 und 30.

Die Menge des Sumpfablaufes ist außerordentlich gering und liegt bei weniger als 10 Gew.-%, oft sogar bei weniger als 5 Gew.-% des zugegeben Acetals, da nahezu das gesamte zugegebene Acetal der Formel II in Wertprodukte gespalten wird und nur ein sehr kleiner Teil der Reaktanten zu schwersiedenden Nebenprodukten reagiert. Auch diese hohe Selektivität stellt einen großen Vorteil des erfindungsgemäßen Verfahrens dar.

Für den Fall, daß die Spaltkolonne keinen Seitenabzug aufweist, besteht das am Kopf der Kolonne erhaltene Destillat im wesentlichen aus den Wertprodukten Prenol, 3-Methyl-2-butenal, Citral und den Citralvorstufen der Formeln IV und V. Diese Mischung kann auf einfache Weise destillativ, beispielsweise durch Destillation in einer weiteren Kolonne, in Prenol und ggf. das aus der Herstellung des Acetals der Formel II stammende unumgesetzte 3-Methyl-2-butenal sowie in Citral und die Citralvorstufen der Formeln IV und V getrennt werden. Es ist auch möglich, die in dieser Mischung enthaltenen Citralvorstufen durch Erhitzen auf Temperaturen zwischen 100 °C und 200 °C in Citral umzulagern und dann das Citral destillativ abzutrennen. Die zurückgewonnenen Verbindungen Prenol und ggf. 3-Methyl-2-butenal können wieder zur Herstellung des Acetals der Formel II verwendet werden.

Mit besonderem Vorteil verwendet man zur Durchführung des erfindungsgemäßen Verfahrens jedoch eine Spaltkolonne mit einem Seitenabzug. In diesem Fall kann auf eine zusätzliche Destillationskolonne verzichtet werden. Der Kopfabzug der Spaltkolonne besteht dann im wesentlichen aus Prenol und ggf. 3-Methyl-2-butenal, die ohne weiteres wieder zur Herstellung des Acetals der Formel II eingesetzt werden können. Der durch den Seitenabzug gewonnene Produktstrom besteht zu mehr als 70 Gew.-% und oft sogar zu mehr als 85 Gew.-% aus Citral und den Citralvorstufen der Formeln IV und V. Die in diesem Produktstrom enthaltenen Citralvorstufen werden auf bekannte Art durch Erhitzten auf Temperaturen zwischen 100 °C und 200 °C in Citral umgelagert.

Mit Hilfe des erfindungsgemäßen Verfahrens ist es möglich, das als Riechstoff und als Zwischenprodukt für Riechstoffe und Vitamine begehrte Citral auch in industriellem Maßstab auf einfache und kostengünstige Weise kontinuierlich und mit ausgezeichneten Ausbeuten herzustellen.

Die folgenden Beispiele sollen das Verfahren erläutern.

### Beispiele 1 bis 3

Die Apparatur bestand aus einem elektrisch beheizten 1-Liter-Kolben mit aufgesetzter Kolonne, die mit einem elektrischen Rückflußteiler (zur Erzielung von einem Rückflußverhältnis von 1:1) ausgestattet war. Die Kolonne war mit einer 1m hohen Sulzer Ex-Packung gefüllt. Zu Beginn des Experimentes wurde der Kolben mit 500 g eines 90 gew.-%-igen Acetals der Formel II gefüllt. In den Beispielen 1 und 3 wurden bis zu 31 ppm Phosphorsäure als Katalysator zugegeben. In Beispiel 2 wurde ohne Zugabe eines Katalysators gearbeitet. Der Kopfdruck der Kolonne betrug zwischen 5 und 60 mbar. Nach Aufheizen des Kolbeninhaltes wurden kontinuierlich zwischen 100 g/h und 200 g/h 3-Methyl-2-buten-diprenylacetal zugepumpt. Die sich bildenden Spaltprodukte (hauptsächlich Prenol, und die Citralvorstufen cis/trans-Prenyl-(3-methyl-butadienyl)-ether der Formel IV und 2,4,4-Trimethyl-3-formyl-1,5-hexadien der Formel V wurden fortlaufend abdestilliert und gesammelt. Das Acetal wurde durch Rückfluß und Regeln der Heizleistung in dem Reaktionskolben zurückgehalten. Nach Ende des Experimentes wurden das Destillat und der Inhalt des Kolbens ausgewogen und gaschromatographisch analysiert. Die Parameter und Ergebnisse der Beispiele 1 bis 3 sind in der Tabelle 1 zusammengestellt.

**Tabelle 1**

| | Beispiel 1 | Beispiel 2 | Beispiel 2 |
|---|---|---|---|
| Zufluß an Acetal [ml/h] | 100 | 100 | 200 |
| Druck am Kolonnenkopf [mbar] | 6 | 60 | 5 |
| Druck im Kolben [mbar] | 20 | 70 | 23 |
| Temperatur im Kolben [°C] | 143 bis 148 | 169 bis 190 | 143 bis 148 |
| Einwaage an Acetal [g] | 507,8 | 503 | 507,3 |
| Konzentration an H₃PO₄ im Kolben [ppm] | 31 | ohne | 27,2 |
| Acetal (Füllung + zugeflossen) [g] | 891,3 | 1107,8 | 1485,2 |
| Zugeflossene Menge an Acetal [g] | 383,5 | 604,8 | 977,9 |
| Destillat [g] | 711,9 | 958,4 | 1174,7 |
| Sumpf nach Versuchsende [g] | 172,2 | 153,3 | 312,7 |
| Umsatz des Acetals | 83,1 % | 97,4 % | 49,8 % |
| Selektivität zu Prenol | 98,7 % | 95,2 % | 92,7 % |
| Selektivität zu Citral und Citralvorstufen | 89,1 % | 96,0 % | 97,8 % |

### Beispiele 4 bis 8

Die Parameter und Ergebnisse der Beispiele 4 bis 8 sind in der Tabelle 2 zusammengestellt. Die Apparatur bestand aus einer Spaltkolonne mit 30 theoretischen Trennstufen. Der Sumpf der Kolonne wurde mit einem Fallfilmverdampfer beheizt. 10 Trennstufen oberhalb des Sumpfes befand sich der Seitenabzug für das Citral und die Citralvorstufen. Das am Kopf der Kolonne kondensierende Destillat wurde gesammelt und ein Teil davon mit einer Pumpe als Rückfluß auf den Kopf der Kolonne zurück gepumpt. Pro Stunde wurden 100 g Acetal der Formel II in den Sumpf der Kolonne zugegeben. Als Katalysator wurde als eine etwa 1-gew.-%ige Lösung von H₃PO₄ in dem Acetal der Formel II in den Sumpf der Kolonne zugegeben. Die Menge an Katalysator-Lösung wurde so bemessen, daß die in der Tabelle angegebene Konzentration an H₃PO₄ im Sumpf der Kolonne erhalten wurde. Die Selektivität zu Prenol war größer als 89,3 %.

**Tabelle 2**

| Beispiel | 4 | 5 | 6 | 7 | 8 |
|---|---|---|---|---|---|
| H₃PO₄-Konzentration [ppm] | 117 | 475 | 332 | 269 | 1044 |
| Temperatur im Sumpf [°C] | 175 | 145 | 155 | 160 | 150 |
| Druck am Kolonnenkopf [mbar] | 50 | 30 | 30 | 30 | 50 |
| Rückflußmenge [g/h] | 968 | 774 | 968 | 774 | 774 |
| Acetalkonz. im Zufluß [kg/kg] | 83,94 % | 76,93 % | 76,93 % | 88,25 % | 85,82 % |
| Sumpfabzug [g/h] | 0,37 | 33,51 | 6,82 | 0,51 | 6,91 |
| Seitenabzug [g/h] | 57,00 | 29,76 | 47,33 | 61,74 | 52,13 |
| Kopfabzug [g/h] | 42,80 | 38,26 | 48,84 | 35,22 | 40,44 |
| Rückflußverhältnis | 23 | 20 | 20 | 22 | 19 |
| Inhalt des Sumpfes [g] | 100 | 100 | 100 | 170 | 170 |
| Umsatz des Acetals | 99,5 % | 67,5 % | 95,3 % | 99,8 % | 99,3 % |
| Selektivität zu Citral und Citralvorstufen | 91,8 % | 96,6 % | 90,0 % | 93,4 % | 97,6 % |

## Patentansprüche

1. Verfahren zur Herstellung von 3,7-Dimethyl-2,6-octadien-1-al (Citral) der Formel I 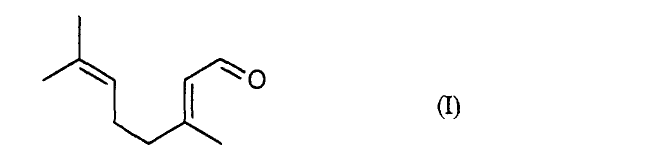 durch thermische Spaltung ggf. in Gegenwart eines sauren Katalysators von 3-Methyl-2-buten-1-al-diprenylacetal der Formel II 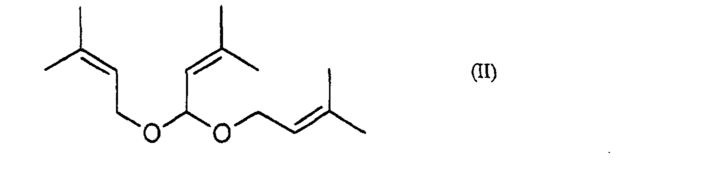 unter Abspaltung von 3-Methyl-2-buten-1-ol (Prenol) der Formel III zu cis/trans-Prenyl-(3-methyl-butadienyl)-ether der Formel IV 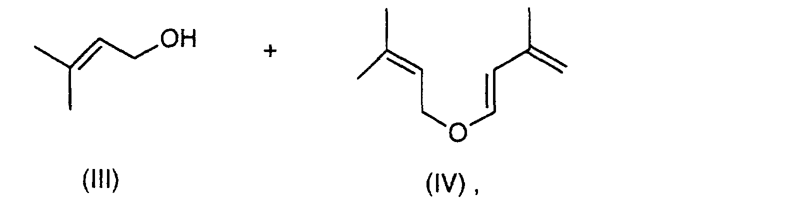 Claisen-Umlagerung des erhaltenen Butadienylethers der Formel IV zu 2,4,4-Trimethyl-3-formyl-1,5-hexadien der Formel V 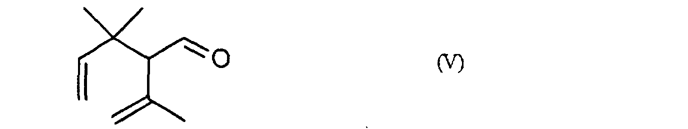 und anschließende Cope-Umlagerung desselben zum Citral der Formel I, **dadurch gekennzeichnet, daß** man sowohl das gebildete Prenol der Formel III, als auch die intermediär gebildeten Zwischenprodukte der Formeln IV und V und das Citral schon während der Umsetzung fortlaufend destillativ aus dem Reaktionsgemisch entfernt und die Zwischenprodukte der Formeln IV und V vor oder nach destillativer Abtrennung von Prenol und ggf. Nebenprodukten durch Erhitzen auf Temperaturen zwischen 100 und 200°C in Citral umlagert.

2. Verfahren gemäß Anspruch 1, **dadurch gekennzeichnet, daß** man die thermische Spaltung des Acetals der Formel II im unteren Teil oder im Sumpf einer Destillationskolonne mit 5 bis 100 theoretischen Trennstufen durchführt.

3. Verfahren gemäß Anspruch 2, **dadurch gekennzeichnet, daß** man das Acetal der Formel II und ggf. den sauren Katalysator in den unteren Teil der Kolonne, in den Sumpf der Kolonne oder in den Verdampfer (2) der Kolonne einbringt.

4. Verfahren gemäß Anspruch 1, **dadurch gekennzeichnet, daß** man die thermische Spaltung des Acetals der Formel II im unteren Teil oder im Sumpf einer Destillationskolonne mit 5 bis 100 theoretischen Trennstufen durchführt, wobei man das Acetal der Formel II durch geeignete Auswahl der Destillationsbedingungen im unteren Teil bzw. im Sumpf der Kolonne hält, das gebildete Citral der Formel I, die intermediär gebildeten Zwischenprodukte der Formeln IV und V und das abgespaltene Prenol der Formel III gemeinsam am Kopf der Kolonne als Kopfstrom gewinnt und das Gemisch aus Citral und den Zwischenprodukten der Formeln IV und V vor oder nach destillativer Abtrennung von Prenol und ggf. Nebenprodukten durch ein beheiztes Verweilzeitrohr (7) führt, in dem die Zwischenprodukte der Formeln IV und V bei Temperaturen von 100 bis 200°C zu Cital umgelagert werden.

5. Verfahren gemäß Anspruch 1, **dadurch gekennzeichnet, daß** man die thermische Spaltung des Acetals der Formel II im unteren Teil (9) oder im Sumpf (10) einer Destillationskolonne (1) mit 5 bis 100 theoretischen Trennstufen durchführt, wobei man das Acetal der Formel II durch geeignete Auswahl der Destillationsbedingungen im unteren Teil (9) bzw. im Sumpf (10) der Kolonne (1) hält, das gebildete Citral der Formel I und die intermediär gebildeten Zwischenprodukte der Formeln IV und V gemeinsam an einem im mittleren Teil der Kolonne angeordneten Seitenabzug (5) flüssig oder dampfförmig abzieht und das abgespaltene Prenol der Formel III am Kopf (8) der Kolonne mit dem Kopfstrom abtrennt.

6. Verfahren gemäß Anspruch 5, **dadurch gekennzeichnet, daß** man das an dem Seitenabzug (5) abgezogene Gemisch aus Citral und den intermediär gebildeten Zwischenprodukten der Formeln IV und V durch ein beheiztes Verweilzeitrohr (7) führt, in dem die Zwischenprodukte der Formeln IV und V bei Temperaturen von 100 bis 200 °C zu Citral umgelagert werden.

7. Verfahren gemäß Anspruch 5, **dadurch gekennzeichnet, daß** man die Herstellung des Citrals kontinuierlich durchführt.

8. Verfahren gemäß Anspruch 5, **dadurch gekennzeichnet, daß** sich der Seitenabzug (5) 2 bis 20 theoretische Trennstufen oberhalb der Zugabestelle (6) für das Acetal der Formel II und 2 bis 80 theoretische Trennstufen unterhalb des Kolonnenkopfes (8) befindet.

9. Verfahren gemäß Anspruch 2, **dadurch gekennzeichnet, daß** der Druck im Sumpf der Kolonne zwischen 1 und 100 mbar beträgt und die Temperatur im Sumpf der Kolonne zwischen 70 und 270 °C beträgt.

10. Verfahren gemäß Anspruch 2, **dadurch gekennzeichnet, daß** das Rücklaufverhältnis am Kopf der Destillationskolonne zwischen 0,5 und 70.

## Claims

1. A process for preparing 3,7-dimethyl-2,6-octadien-1-al (citral) of the formula I 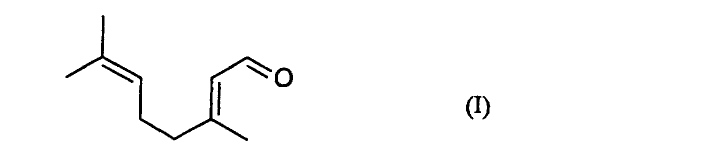 by thermal cleavage, in the presence or absence of an acid catalyst, of 3-methyl-2-buten-1-al diprenyl acetal of the formula II 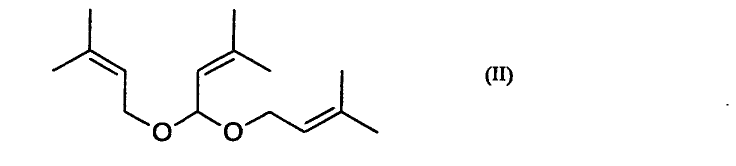 with elimination of 3-methyl-2-buten-1-ol (prenol) of the formula III to give cis/trans-prenyl 3-methylbutadienyl ether of the formula IV 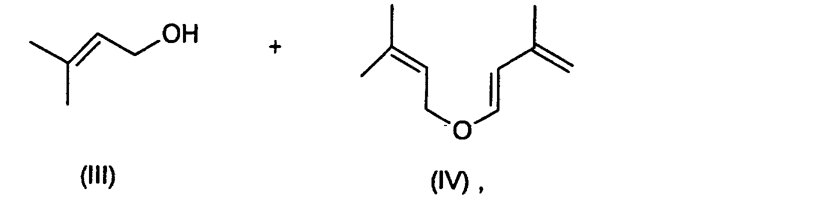 Claisen rearrangement of the resultant butadienyl ether of the formula IV to give 2,4,4-trimethyl-3-formyl-1,5-hexadiene of the formula V 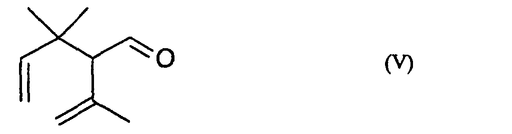 and subsequent Cope rearrangement of the same to give citral of the formula I,
which comprises continuously removing by distillation from the reaction mixture not only the prenol formed of the formula III, but also the intermediates of the formulae IV and V and the citral, even during the reaction, and rearranging the intermediates of the formulae IV and V by heating them to from 100 to 200°C to form citral before or after removal of prenol and possibly byproducts by distillation.

2. A process as claimed in claim 1, wherein the thermal cleavage of the acetal of the formula II is carried out in the lower part or in the bottom of a distillation tower having from 5 to 100 theoretical plates.

3. A process as claimed in claim 2, wherein the acetal of the formula II, with or without the acid catalyst, is introduced into the lower part of the tower, into the bottom of the tower or into the tower evaporator (2).

4. A process as claimed in claim 1, wherein the thermal cleavage of the acetal of the formula II is carried out in the lower part or in the bottom of a distillation tower having from 5 to 100 theoretical plates, the acetal of the formula II being kept by suitable choice of distillation conditions in the lower part, or in the bottom, of the tower, the citral formed of the formula I, the intermediates formed of the formulae IV and V and the eliminated prenol of the formula III being taken off together at the top of the tower as overhead stream and the mixture of citral and the intermediates of the formulae IV and V, before or after removing prenol, and possibly byproducts, by distillation, then being passed through a heated delay tube (7) in which the intermediates of the formulae IV and V are rearranged at from 100 to 200°C to give citral.

5. A process as claimed in claim 1, wherein the thermal cleavage of the acetal of the formula II is carried out in the lower part (9) or in the bottom (10) of a distillation tower (1) having from 5 to 100 theoretical plates, the acetal of the formula II being kept in the lower part (9) or in the bottom (10) of the tower (1) by suitable choice of the distillation conditions, the citral formed of the formula I and the intermediates formed of the formulae IV and V being taken off together in the liquid state or vapor state at a sidestream takeoff (5) disposed in the central part of the tower and the prenol eliminated of the formula III being separated off at the top (8) of the tower with the overhead stream.

6. A process as claimed in claim 5, wherein the mixture of citral and the intermediates formed of the formulae IV and V which is taken off at the sidestream takeoff (5) is passed through a heated delay tube (7) in which the intermediates of the formulae IV and V are rearranged to form citral at from 100 to 200 °C.

7. A process as claimed in claim 5, wherein citral is prepared continuously.

8. A process as claimed in claim 5, wherein the sidestream takeoff (5) is situated from 2 to 20 theoretical plates above the feed point (6) for the acetal of the formula II and from 2 to 80 theoretical plates below the tower top (8).

9. A process as claimed in claim 2, wherein the pressure in the bottom of the tower is from 1 to 100 mbar and the temperature in the bottom of the tower is from 70 to 270 °C.

10. A process as claimed in claim 2, wherein the reflux ratio at the top of the distillation tower is from 0.5 to 70.

## Revendications

1. Procédé pour la préparation de 3,7-diméthyl-2,6-octadiène-1-al (citral) de formule I 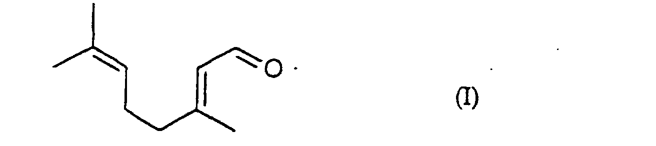 par coupure thermique, éventuellement en présence d'un catalyseur acide, de 3-méthyl-2-butène-1-aldiprénylacétal de formule II 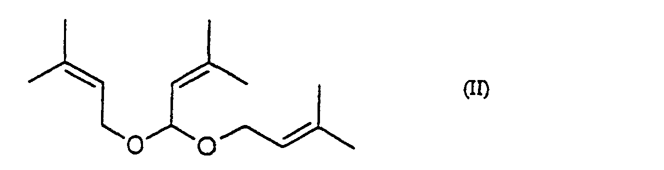 avec élimination de 3-méthyl-2-butène-1-ol (prénol) de formule III pour former l'éther cis/transprényl-(3-méthyl-butadiénylique) de formule IV 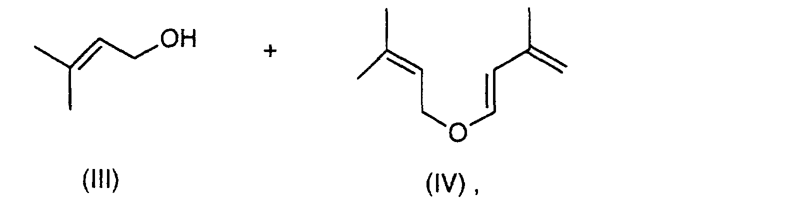 transposition de Claisen de l'éther butadiénylique obtenu de formule IV pour former le 2,4,4-triméthyl-3-formyl-1,5-hexadiène de formule V 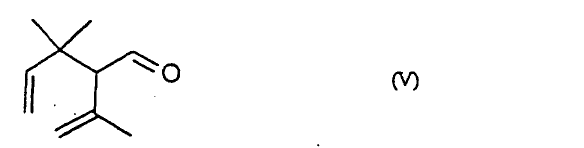 et ensuite transposition de Cope de celui-ci pour former le citral de formule I,
**caractérisé par le fait qu'**on sépare du mélange réactionnel, en continu par distillation, aussi bien le prénol de formule III formé que les produits intermédiaires de formules IV et V formés à titre intermédiaire et le citral, et on convertit en citral les produits intermédiaires de formules IV et V avant ou après séparation par distillation du prénol et éventuellement de sous-produits, par chauffage à des températures comprises entre 100 et 200°C.

2. Procédé selon la revendication 1, **caractérisé par le fait qu'**on conduit la coupure thermique de l'acétal de formule II dans la partie inférieure ou dans le limon d'une colonne de distillation ayant 5 à 100 étages de séparation théoriques.

3. Procédé selon la revendication 2, **caractérisé par le fait qu'**on introduit l'acétal de formule II et éventuellement le catalyseur acide dans la partie inférieure de la colonne, dans le limon de la colonne ou dans l'évaporateur (2) de la colonne.

4. Procédé selon la revendication 1, **caractérisé par le fait qu'**on effectue la coupure thermique de l'acétal de formule II dans la partie inférieure ou dans le limon d'une colonne de distillation ayant 5 à 100 étages de séparation théoriques, tandis qu'on maintient l'acétal de formule II par un choix approprié des conditions de distillation dans la partie inférieure ou respectivement dans le limon de la colonne, on recueille le citral de formule I formé, les produits intermédiaires de formules IV et V formés à titre intermédiaire et le prénol de formule III séparé, ensemble en tête de colonne sous la forme d'un courant de tête, et on entraîne le mélange de citral et des produits intermédiaires de formules IV et V avant ou après séparation par distillation du prénol et éventuellement des sous-produits, par une conduite à temps de séjour (7) chauffée; dans laquelle les produits intermédiaires de formules IV et V sont convertis en citral à des températures de 100 à 200°C.

5. Procédé selon la revendication 1, **caractérisé** part le fait qu'on conduit la coupure thermique de l'acétal de formule II dans la partie inférieure (9) ou dans le limon (10) d'une colonne de distillation (1) ayant 5 à 100 étages de séparation théoriques, tandis qu'on maintient l'acétal de formule II, par un choix approprié des conditions de distillation, dans la partie inférieure (9) ou respectivement dans le limon (10) de la colonne (1), on extrait sous forme de liquide ou de vapeur le citral de formule I formé et les produits intermédiaires de formules IV et V formés à titre intermédiaire, ensemble par une évacuation latérale (5) située dans la partie médiane de la colonne, et on sépare avec le courant de tête le prénol de formule III formé en tête (8) de la colonne.

6. Procédé selon la revendication 5, **caractérisé par le fait qu'**on envoie le mélange, soutiré par l'évacuation latérale (5), de citral et des produits intermédiaires de formules IV et V formés à titre intermédiaire, par l'intermédiaire d'une conduite à temps de séjour chauffée (7), dans laquelle les produits intermédiaires de formules IV et V sont convertis en citral à des températures de 100 à 200°C.

7. Procédé selon la revendication 5, **caractérisé par le fait qu'**on conduit la préparation du citral en continu.

8. Procédé selon la revendication 5, **caractérisé par le fait que** l'évacuation latérale (5) se situe 2 à 20 étages de séparation théoriques au-dessus de l'emplacement d'introduction (6) pour l'acétal de formule II et 2 à 80 étages de séparation théoriques en dessous de la tête de colonne (8).

9. Procédé selon la revendication 2, **caractérisé par le fait que** la pression dans le limon de la colonne se situe entre 1 et 100 mbar et la température dans le limon de la colonne se situe entre 70 et 270°C.

10. Procédé selon la revendication 2, **caractérisé par le fait que** la proportion de reflux en tête de la colonne de distillation vaut entre 0,5 et 70.
